# EUROPEAN PATENT APPLICATION

(11) **EP 3 769 737 A1**
(43) Date of publication of application: **27.01.2021**
(21) Application number: 19772005.5
(22) Date of filing: 12.03.2019
(51) Int. Cl.: A61G 7/008, A61G 7/018, A61G 7/005, G16H 20/30

(54) **BED WITH ANGULAR POSITION CONTROL AND METHOD FOR CHANGING THE ANGULAR POSITION**

(30) Priority: 19.03.2018 ES 201830263
(71) Applicant: Lluis Creus, S.L., 08918 Badalona (ES)
(72) Inventor: BARRANCA TEIXIDÓ, Miquel, 08918 Badalona (ES)
(74) Representative: Espiell Gomez, Ignacio
(86) International application number: PCT/ES2019/070165
(87) International publication number: WO 2019/180287

(57) **Abstract**

The invention relates to a bed with angular position control with automatic angular position control, with a lower frame (2) and an upper frame (4) pivoting about a shaft (5) by means of a mechanism (7) automatically actuated through an electronic device (8) with a programmable microprocessor which can be connected to an external switch (9) that is operated without using hands, to sensors (10) incorporated in the bed (1) and/or in accessory elements (11), and has a wireless communication module such as a Wi-Fi, Bluetooth, or radio communication module, for local or remote connection. The mechanism (7) comprises a single actuator, preferably a linear actuator (71), in the central area of the structure with one end fastened in the lower frame (2) and the other end fastened to one side of the upper frame (4).

## Description

### OBJECT OF THE INVENTION

As stated in the title of the present specification, the invention relates to a bed with angular position control, as well as to a method for changing the angular position, providing the intended function thereof with advantages and novel features that are described in detail below and constitute a significant improvement over the current state of the art.

The object of the present invention is a bed with angular position control of the type intended for achieving a change in the maximum pressure area or pressure distribution of bedridden users who are unable to change their own positions by means of the invention which generates a side tilting movement of the structure on which the bed is supported. The bed has, among other innovations, the particularity of having a programmable electronic device providing automated control for actuating the changing in angular position, providing significant advantages with respect to what is known up until now on the market.

### FIELD OF APPLICATION OF THE INVENTION

The field of application of the present invention is comprised within the sector of the industry dedicated to the manufacturing of rest elements, particularly beds, covering at the same time the field of medical and orthopaedic apparatuses and devices, and similar ones for technical assistance.

### BACKGROUND OF THE INVENTION

As is known, people with mobility issues and/or diseases preventing them from moving around by themselves, for example, those affected by spinal cord injury, multiple sclerosis (MS), amyotrophic lateral sclerosis (ALS), cerebrovascular accident (CVA), and the elderly, require periodic and frequent help to change their position in bed because, otherwise, they will be plagued by the onset of pressure ulcers.

To help prevent said pressure from causing such ulcers and/or to alleviate the existence thereof upon their onset, the so-called beds with angular position control are known on the market; said beds, installed on a double frame structure, incorporate a mechanism which enables it to render a side rocking movement thereto, causing a variation in the pressure distribution (maximum pressure point) by moving the centre of gravity of the user. The relative position of the patient with respect to the mattress usually does not vary.

However, said type of bed has certain aspects that can be improved including, most significantly, the difficulty for the user him/herself to handle same, given that normally the ability to of the user to move, and accordingly to autonomously operate and handle the movements of the bed, is very limited.

Therefore, the objective of the present invention focuses on developing a novel bed with angular position control with automated operations in order to prevent as much as possible the aforementioned drawbacks in terms of the difficulty this type of bed entails for the users thereof. Another objective of the invention is a method for changing the angular position of the bed.

Moreover, and as reference to the current state of the art, it must be indicated that, although other beds with angular position control are known as have been indicated, the applicant at least is unaware of the existence of any bed having technical and structural features that are identical or similar to those herein claimed.

### DESCRIPTION OF THE INVENTION

Thus, the bed with automatic angular position control proposed by the invention amounts to a noteworthy novelty within its field of application, the characterising details that set it apart being duly recorded in the claims included at the end of the present specification.

Specifically, as indicated above, the invention proposes a bed with angular position control of the type presenting a side tilting movement of the structure on which it is supported, for the purpose of achieving a change in the pressures of the intended user, so as to change the maximum pressure point over time and thereby prevent the generation of sores, while at the same time improving the rest and circulation of said user who is usually someone who is unable to change their own position, said bed presenting, among other particularities, the particularity of having a programmable electronic device providing automated control for actuating the tilting movement.

Said device enables a series of programmes to be incorporated so that the bed moves in an autonomous manner and gradually changes the pressures of the user in a comfortable and quiet manner, preventing a caretaker and a helper from having to perform such changes by means of handling same, in both the prevention and treatment of pressure ulcers.
This automated programmable control therefore provides the following advantages:
- It enables a continuous tilting movement of the bed to be programmed.
- It enables different movement parameters to be chosen, such as the maximum angle of inclination, speed of movement, starting point.
- It enables a delay to be established so that the movement will start after x minutes;
- Additionally, the electronic control device may incorporate at least one inlet port for connecting a switch from those that are known so that people with disabilities may use same without using their hands, for example, a switch that is operated using sight, sound, breathing, blowing, scanning, or others; and
- Optionally, the electronic control device of the bed also has one or more inlets for connection to different sensors, strategically incorporated in different parts of the bed or accessories.
- Optionally, the electronic control device comprises a wireless communication module, such as a Wi-Fi, Bluetooth, or radio communication module, such that it can be used as a receiver for receiving variables from the user or bed which are detected by said sensors and sending them to a local or remote database, as well as for controlling the bed from other devices, for example from a PC, an electronic tablet, or a mobile phone, or a home automation system. This enables a caretaker to remotely use and consult data relating to the bed without having to disturb the user, or a caretaker may even be able to remotely consult data such as the weight or pressure history and movements of both the bed and the user.

Preferably, the sensors that are, for example, incorporated in the bed and connected to the electronic control device are:
- bed-abandonment sensor and/or presence sensor,
- load cell weight reader,
- open rail detector,
- actuator position sensor,
- pressure sensor,
- light detector.

It must be pointed out that, particularly, a preferred option of the bed of the invention contemplates the incorporation of a pressure blanket wherein several pressure sensors detecting the weight of a body on the bed are strategically distributed.

In any case, said sensors will be connected to the electronic device. The electronic device can be programmed so that it communicates the variables captured by the sensors to the caretaker wirelessly.

Furthermore, all the data can be archived as part of user history in the device itself and can even be sent to the caretaker.

The electronic control device can thereby further interact automatically with the sensors such that, if the sensors capture certain previously programmed variables, the device can send the signal to the mechanism determining the movement of the bed so that it moves a certain manner.

For example, a pressure sensor (pressure blanket) can capture information relating to whether the user has spent a long time in one same position, and accordingly whether a part of their body is receiving a high pressure for too long. The device can be programmed so that, in such case, it orders the actuator mechanism of the bed to perform a movement in order to change the high pressure point with the user.

Another example is the blocking of the movement for changing the angular position if a sensor of the bed detects that the rails are open, thereby preventing the movement from causing the user from being able to fall off the bed.

In this sense, a method for changing the angular position of the bed with the following steps is provided.
- Programming the movement for changing the angular position
- The sensors capturing bed and/or user variables
- Checking the variables
- Carrying out the movement for changing the angular position In a preferred embodiment, the process has the following steps
- Programming the movement for changing the angular position
- A sensor capturing if the rails are open or closed
- Checking the variables
- Carrying out the movement for changing the angular position only if the rails are closed.

In another preferred embodiment, the process has the following steps
- Programming the movement for changing the angular position
- A pressure blanket capturing the maximum pressure point and the time the maximum pressure is at said point
- Checking if the time values are above a previously programmed maximum time
- Carrying out the movement for changing the angular position only if the maximum time has been exceeded.

In another preferred embodiment, the process has the following steps
- Programming the movement for changing the angular position
- A sensor (for example, a pulse sensor) capturing user variables in order to determine if the user is asleep
- Determining if the captured variables meet the previously established requirements in order to consider that the user is asleep
- Carrying out the movement for changing the angular position only if the user is asleep.

Moreover, the side tilting movement of the bed, which bed, as is known, has a support structure comprising a lower frame, usually equipped with wheels, and an upper frame, wherein the bed base on which the mattress is arranged is integrated, said upper frame tilting laterally towards either side, and another pivoting about a shaft which is in turn coupled to an intermediate support which links it with the lower frame, is determined by a mechanism preferably comprising a single actuator, specifically a linear actuator, arranged in the central area of the bed structure such that one end is fastened to the lower frame and the other end fastened to one side of the upper frame, determining when it extends and retracts the upward and downward movement of said side, and therefore the tilting movement of said upper frame.

Preferably, this linear actuator is a direct-drive actuator, although it may optionally comprise a connecting rod interposed in the fastening thereof to the upper frame.

However, the invention provides other optional embodiment variants, for example, variants wherein the mechanism comprises an arch on and about which the shaft of the upper frame slides and rotates, or wherein the mechanism comprises a motor, the transmission shaft of which is linked to the shaft of the upper frame in order to cause it to rotate several degrees towards one side or the other.

The bed with automatic angular position control described therefore consists of an innovative structure with features heretofore unknown for its intended purpose, reasons which, taken together with its usefulness, provide it with sufficient grounds for obtaining the requested exclusivity privilege.

### DESCRIPTION OF THE DRAWINGS

To complete the description provided herein, and for the purpose of helping to make the features of the invention more readily understandable, this description is accompanied by drawings constituting an integral part of the same, which by way of illustration and not limitation represents the following:

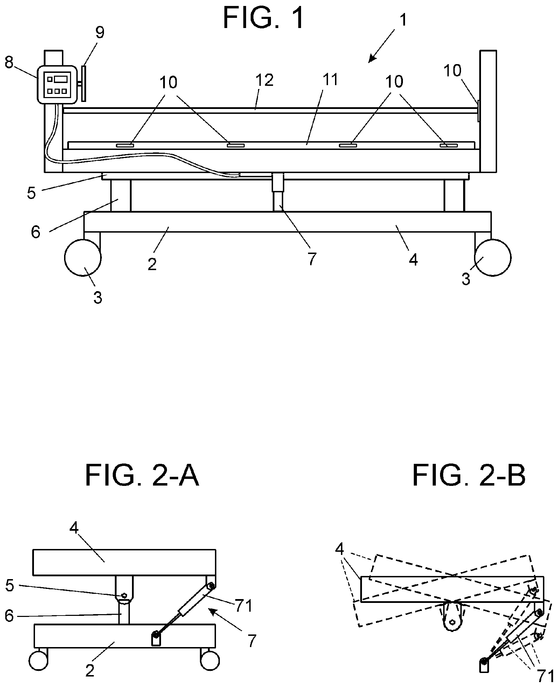

Figure 1 shows a schematic side elevational view of an example of the bed with angular position control, object of the invention, with the main parts and elements it comprises being observed;
Figures 2-A and 2-B show respective schematic front elevational views of the essential parts of the bed with angular position control of the invention, in an example thereof in which the mechanism actuating the movement is formed by a linear direct-drive actuator, with Figure 2-A showing the configuration and arrangement thereof on the lower frame and Figure 2-B showing the movement it renders to the upper frame or bed base;

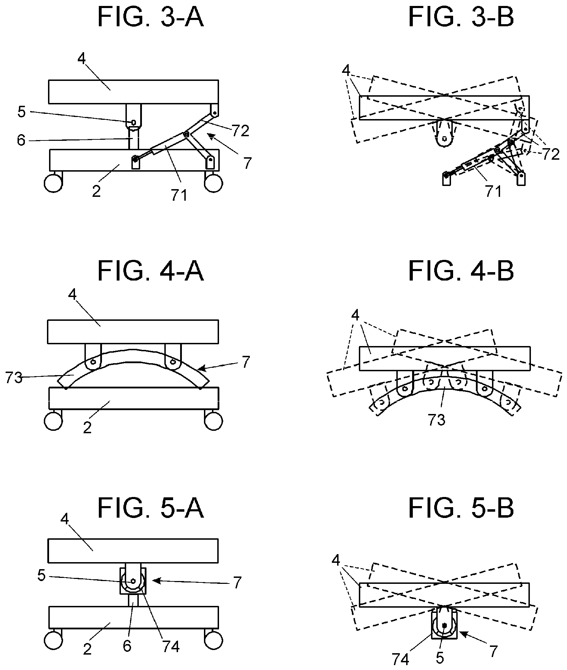

Figures 3-A and 3-B show respective schematic front elevational views of the essential parts of the bed with angular position control of the invention, in this case an example wherein the mechanism is formed by a linear actuator with a connecting rod, with Figure 3-A showing the configuration and arrangement thereof on the lower frame and Figure 3-B showing the movement which it renders to the upper frame or bed base;
Figures 4-A and 4-B show respective schematic front elevational views of the essential parts of the bed with angular position control of the invention, in this case an example wherein the mechanism contemplates an arch-shaped track, with Figure 4-A showing the configuration and arrangement thereof on the lower frame and Figure 4-B showing the movement which it renders to the upper frame or bed base; and
Figures 5-A and 5-B show respective schematic front elevational views of the essential parts of the bed with angular position control of the invention, in another example wherein the mechanism is a motor, with Figure 5-A showing the configuration and arrangement thereof on the lower frame and Figure 5-B showing the movement which it renders to the upper frame or bed base;

### PREFERRED EMBODIMENT OF THE INVENTION

In light of the aforementioned figures, and in accordance with the adopted numbering, one may observe therein an exemplary embodiment of the bed with angular position control of the invention, which comprises the parts and elements indicated and described in detail below.

In this sense, in reference to Figure 1, it can be seen how the bed (1) of the invention is configured from a support structure formed by a lower frame (2) usually equipped with wheels (3), and an upper frame (4) integrating the bed base, which upper frame pivots about a central shaft (5) coupled to an intermediate support (6) linking it with the lower frame (2) and arranged in the longitudinal direction with respect to the bed, such that the upper frame (4) presents a movement for changing the angular position, i.e., tilting towards either side, which is determined by a mechanism (7) which, in an innovative manner, is automatically actuated through an electronic device (8) with a programmable microprocessor, with which it is connected in a wired or wireless manner, which enables the movement of the bed to be controlled autonomously as programmed, so that it performs different actions, for example, a continuous movement, certain maximum angle of inclination, speed of movement, delay of the starting point for the start of the movement, etc.

The electronic device (8) itself incorporates at least one inlet port for connecting an external switch (9) that is operated without using hands, for example, by using sight, sound, breathing, blowing, scanning, or others.

Preferably, the electronic device (8) has one or more inlets for connection to sensors (10) incorporated in the bed (1) and/or in accessory elements (11) thereof, for example, a bed-abandonment sensor and/or presence sensor, load cell weight reader, open rail detector, actuator position sensor, pressure sensor, light detector, such that said device receives and records different bed and/or user variables that can be taken into account for the programming thereof.

In the main embodiment, the bed (1) comprises an accessory element (11) consisting of a pressure blanket provided with various pressure sensors (10) strategically distributed therein, detecting the weight of a body thereon in order to enable the electronic device (8) to be programmed such that it actuates the mechanism (7) after a specific time period without variation in the received pressure.

The bed (1) also preferably incorporates an open rail detection sensor (10) in order to programme the blocking of the movement for changing the angular position of the mechanism (7) in the electronic device (8) with the rail (12) being open.

In any case, advantageously, the electronic device (8) also incorporates a wireless communication module, such as a Wi-Fi, Bluetooth, or radio communication module, for the connection thereof with other local or remote devices, for example a PC, an electronic tablet, or a mobile phone, in order to access the information it records and/or remotely control the operation thereof.

In turn, in reference to figure 2-A, it can be seen how the mechanism (7) determining the angular position movement of the bed (1) preferably comprises a single actuator, which consists of a linear direct-drive actuator (71) arranged in the central area of the support structure of the bed (1) with one end fastened in the lower frame (2) and the other end fastened to one side of the upper frame (4), determining, as shown in figure 2-B, when it extends and retracts the upward and downward movement of said side, and therefore the tilting movement of said upper frame (4).

Figures 3-A and 3-B show another embodiment option wherein the linear actuator (71) incorporates a connecting rod (72) interposed in the fastening thereof to the upper frame (4).

In turn, Figures 4-A and 4-B show another alternative embodiment option of the bed (1) wherein the mechanism (7) comprises an arch-shaped track (73) along which the upper frame (4) slides in rotation.

Lastly, Figures 5-A and 5-B show another embodiment option of the bed (1) wherein the mechanism (7) comprises a motor (74) associated with the shaft (5) about which the upper frame (4) pivots.

Having sufficiently described the nature of the present invention, as well as the ways of implementing it, it is not considered necessary to extend its explanation for any expert in the state of the art to understand its scope and the advantages which derive from it, specifying that, within its essential nature, it can be carried out in other embodiments that differ in detail from the one provided by way of example, and which are also covered by the requested protection, provided that they do not alter, change or modify its fundamental principle.

## Claims

1. A bed with angular position control with automatic angular position control configured from a support structure formed by a lower frame (2) and an upper frame (4) pivoting with a tilting movement to either side determined by an actuator mechanism, wherein said actuator mechanism (7) is automatically actuated through an electronic device (8) with a programmable microprocessor, with which it is connected in a wired or wireless manner, and having one or more inlets for connection to sensors (10) incorporated in the bed (1) and/or in accessory elements (11) thereof, from which it receives and records different variables for the programming thereof, **characterised in that** the sensors (10) include pressure sensors detecting the maximum pressure point and the actuator mechanism (7) is configured to be activated if the time the maximum pressure is at one same point exceeds a previously programmed maximum time.

2. The bed with angular position control with automatic angular position control according to claim 1, **characterised in that** the electronic device (8) incorporates at least one inlet port wherein an external switch (9) is connected that is operated without using hands, for example, by using sight, sound, breathing, blowing, scanning, or others.

3. The bed with angular position control with automatic angular position control according to claim 1, **characterised in that** the sensors (10) incorporate a bed-abandonment sensor and/or presence sensor.

4. The bed with angular position control with automatic angular position control according to claim 1 or 3, **characterised in that** said sensors (10) incorporate a load cell weight reader.

5. The bed with angular position control with automatic angular position control according to claim 1, 3, or 4, **characterised in that** said sensors (10) incorporate an open rail detector.

6. The bed with angular position control with automatic angular position control according any of claims 1, 3 to 5, **characterised in that** said sensors (10) incorporate an actuator position sensor.

7. The bed with angular position control with automatic angular position control according to any of claims 1, 3 to 6, **characterised in that** said sensors (10) incorporate a light detector.

8. The bed with angular position control with automatic angular position control according to claim 1, **characterised in that** said bed (1) incorporates an accessory element (11) consisting of a pressure blanket equipped with several pressure sensors (10) distributed therein.

9. The bed with angular position control with automatic angular position control according to claim 1, **characterised in that** the electronic device (8) comprises a wireless communication module, such as a Wi-Fi, Bluetooth, or radio communication module, for the connection thereof with other local or remote devices, such as a PC, an electronic tablet, or a mobile phone, in order to access the information it records and/or remotely control the operation thereof.

10. The bed with angular position control with automatic angular position control according to claim 1, **characterised in that** the mechanism determining the movement for changing the angular position of the bed (1) comprises a single actuator (7).

11. The bed with angular position control with automatic angular position control according to claim 12, **characterised in that** the actuator mechanism (7) is a linear actuator (71) arranged in the central area of the support structure of the bed (1) with one end fastened in the lower frame (2) and the other end fastened to one side of the upper frame (4).

12. The bed with angular position control with automatic angular position control according to claim 13, **characterised in that** the linear actuator (71) is a linear direct-drive actuator.

13. The bed with angular position control with automatic angular position control according to claim 13, **characterised in that** the linear actuator (71) incorporates a connecting rod (72) interposed in the fastening thereof to the upper frame (4).

14. The bed with angular position control with automatic angular position control according to claim 1 or 2, **characterised in that** the actuator mechanism (7) comprises an arch-shaped track (73) along which the upper frame (4) slides.

15. The bed with angular position control with automatic angular position control according to claims 1, 10, and 14, **characterised in that** the actuator mechanism (7) comprises a motor (74) associated with a shaft (5) about which the upper frame (4) pivots.

16. A method for changing the angular position of a bed with angular position control according to any of the preceding claims, **characterised in that** it comprises the following steps
• The user programming a movement for changing the angular position in the electronic device (8) with a programmable microprocessor
• The sensors connected to the electronic device (8) capturing bed and/or user variables, including the maximum pressure point in the body of the user and the time the maximum pressure is at said point
• The electronic device (8) with a programmable microprocessor automatically checking the variables, including if the time values are above a previously programmed maximum time
• The mechanism (7) carrying out the movement for changing the angular position if the maximum time has been exceeded.

17. The method for changing the angular position of a bed with angular position control according to claim 16, **characterised in that** it comprises the following steps
• The user programming a movement for changing the angular position in the electronic device (8) with a programmable microprocessor
• A sensor capturing if the rails are open or closed
• the electronic device (8) with a programmable microprocessor automatically checking the variables in order to determine if the rails are open or closed
• Carrying out the movement for changing the angular position only if the rails are closed.

18. The method for changing the angular position of a bed with angular position control according to claim 16, **characterised in that** it comprises the following steps
• The user programming a movement for changing the angular position in the electronic device (8) with a programmable microprocessor
• A sensor capturing user variables in order to determine if the user is asleep
• Determining if the captured variables meet the previously established requirements in order to consider that the user is asleep
• Carrying out the movement for changing the angular position only if the user is asleep.

## Amended claims

### Amended claims under Art. 19.1 PCT

1. A bed with angular position control with automatic angular position control configured from a support structure formed by a lower frame (2) and an upper frame (4) pivoting with a tilting movement to either side determined by an actuator mechanism, wherein said actuator mechanism (7) is automatically actuated through an electronic device (8) with a programmable microprocessor, with which it is connected in a wired or wireless manner, and having one or more inlets for connection to sensors (10) incorporated in the bed (1) and/or in accessory elements (11) thereof, from which it receives and records different variables for the programming thereof, **characterised in that** the sensors (10) include pressure sensors detecting the maximum pressure point and the actuator mechanism (7) is configured to be activated if the time the maximum pressure is at one same point exceeds a previously programmed maximum time.

2. The bed with angular position control with automatic angular position control according to claim 1, **characterised in that** the electronic device (8) incorporates at least one inlet port wherein an external switch (9) is connected that is operated without using hands, for example, by using sight, sound, breathing, blowing, scanning, or others.

3. The bed with angular position control with automatic angular position control according to claim 1, **characterised in that** the sensors (10) incorporate a bed-abandonment sensor and/or presence sensor.

4. The bed with angular position control with automatic angular position control according to claim 1 or 3, **characterised in that** said sensors (10) incorporate a load cell weight reader.

5. The bed with angular position control with automatic angular position control according to claim 1, 3, or 4, **characterised in that** said sensors (10) incorporate an open rail detector.

6. The bed with angular position control with automatic angular position control according any of claims 1, 3 to 5, **characterised in that** said sensors (10) incorporate an actuator position sensor.

7. The bed with angular position control with automatic angular position control according to any of claims 1, 3 to 6, **characterised in that** said sensors (10) incorporate a light detector.

8. The bed with angular position control with automatic angular position control according to claim 1, **characterised in that** said bed (1) incorporates an accessory element (11) consisting of a pressure blanket equipped with several pressure sensors (10) distributed therein.

9. The bed with angular position control with automatic angular position control according to claim 1, **characterised in that** the electronic device (8) comprises a wireless communication module, such as a Wi-Fi, Bluetooth, or radio communication module, for the connection thereof with other local or remote devices, such as a PC, an electronic tablet, or a mobile phone, in order to access the information it records and/or remotely control the operation thereof.

10. The bed with angular position control with automatic angular position control according to claim 1, **characterised in that** the mechanism determining the movement for changing the angular position of the bed (1) comprises a single actuator (7).

11. The bed with angular position control with automatic angular position control according to claim 12, **characterised in that** the actuator mechanism (7) is a linear actuator (71) arranged in the central area of the support structure of the bed (1) with one end fastened in the lower frame (2) and the other end fastened to one side of the upper frame (4).

12. A method for changing the angular position of a bed with angular position control according to any of the preceding claims, **characterised in that** it comprises the following steps
• The user programming a movement for changing the angular position in the electronic device (8) with a programmable microprocessor
• The sensors connected to the electronic device (8) capturing bed and/or user variables, including the maximum pressure point in the body of the user and the time the maximum pressure is at said point
• The electronic device (8) with a programmable microprocessor automatically checking the variables, including if the time values are above a previously programmed maximum time
• The mechanism (7) carrying out the movement for changing the angular position if the maximum time has been exceeded.

13. The method for changing the angular position of a bed with angular position control according to claim 12, **characterised in that** it comprises the following steps
• The user programming a movement for changing the angular position in the electronic device (8) with a programmable microprocessor
• A sensor capturing if the rails are open or closed
• the electronic device (8) with a programmable microprocessor automatically checking the variables in order to determine if the rails are open or closed
• Carrying out the movement for changing the angular position only if the rails are closed.

14. The method for changing the angular position of a bed with angular position control according to claim 12, **characterised in that** it comprises the following steps
• The user programming a movement for changing the angular position in the electronic device (8) with a programmable microprocessor
• A sensor capturing user variables in order to determine if the user is asleep
• Determining if the captured variables meet the previously established requirements in order to consider that the user is asleep
• Carrying out the movement for changing the angular position only if the user is asleep.
